# EUROPEAN PATENT APPLICATION

(11) **EP 2 628 732 A1**
(43) Date of publication of application: **21.08.2013**
(21) Application number: 12156160.9
(22) Date of filing: 20.02.2012
(51) Int. Cl.: C07D 239/48, A61K 31/505, A61P 31/18

(54) **Novel crystalline form of rilpivirine hydrochloride**

(71) Applicant: Sandoz AG, 4056 Basel (CH)
(72) Inventor: The designation of the inventor has not yet been filed
(74) Representative: Preißinger, Franziska

(57) **Abstract**

The present invention relates to a novel crystalline form of Rilpivirine hydrochloride and to methods for its preparation. Furthermore the present invention relates to the use of the novel crystalline form for the preparation of a medicament. In addition the present invention relates to pharmaceutical compositions comprising an effective amount of the novel crystalline form of Rilpivirine hydrochloride and to methods of preparing the same. Finally the present invention relates to pharmaceutical combinations comprising the novel crystalline form of Rilpivirine hydrochloride and additional therapeutic agents.

## Description

### FIELD OF THE INVENTION

The present invention relates to a novel crystalline form of Rilpivirine hydrochloride and to methods for its preparation. Furthermore the present invention relates to the use of the novel crystalline form for the preparation of a medicament. In addition the present invention relates to pharmaceutical compositions comprising an effective amount of the novel crystalline form of Rilpivirine hydrochloride and to methods of preparing the same. Finally the present invention relates to pharmaceutical combinations comprising the novel crystalline form of Rilpivirine hydrochloride and additional therapeutic agents.

### BACKGROUND OF THE INVENTION

Rilpivirine hydrochloride, 4-[[4-[[4-(2-Cyanoethenyl)-2,6-dimethylphenyl]amino]-2-pyrimidinyl]amino]benzonitrile monohydrochloride, is a non-nucleoside reverse transcriptase inhibitor (NNRTI) of human immunodeficiency virus type 1 (HIV-1) and indicated for the treatment of HIV-1 infection in treatment-naïve adult patients in combination with other antiretroviral agents. The product received marketing approval in the US (brand name Edurant) and is represented by the following general formula (I):

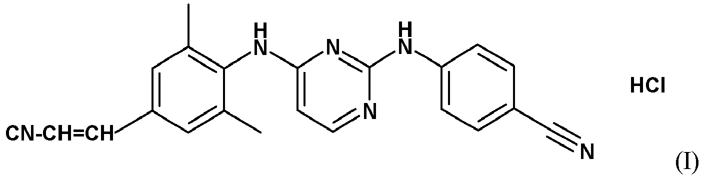

EP1419152 B1 claims amongst others Rilpivirine base and Rilpivirine hydrochloride per se as well as pharmaceutical compositions comprising the same. However, only concrete examples for preparing Rilpivirine base are given in said patent but no concrete examples describing the production of the hydrochloride salt are provided.

EP1632232 B1 claims amongst others a solid pharmaceutical composition comprising crystalline forms A, B, C or D of Rilpivirine hydrochloride. In addition said patent claims a process for the production of Rilpivirine hydrochloride by reacting Rilpivirine base with hydrochloric acid in the presence of a suitable acid, such as acetic acid.

Polymorphism is a phenomenon relating to the occurrence of different crystal forms for one molecule. There may be several different crystalline forms for the same molecule with distinct crystal structures and varying in physical properties like melting point, XRPD pattern and FTIR spectrum. These polymorphs are thus distinct solid forms which share the molecular formula of the compound from which the crystals are made up, however they may have distinct advantageous physical properties such as e.g. chemical stability, physical stability, hygroscopicity, solubility, dissolution rate, bioavailability, etc.

The bioavailability of a compound intended to be administered orally, is dependent on the solubility of the compound in aqueous systems, such as water, as well as the permeability of the compound as mentioned in EP1632232 B1. It is known to the person skilled in the art that the solubility of a crystalline solvate form in the solvent, which is incorporated in this form, is smaller than the solubility of a non-solvated form of the same compound. This means that the solubility of a hydrate - wherein water is the incorporated solvent - in water is thus smaller than that of a corresponding non-hydrated form. For example, hydrates are known to be less soluble in aqueous systems than anhydrous forms of the same compound. Hence anhydrous forms of Rilpivirine hydrochloride are preferred over hydrated forms. Rilpivirine hydrochloride form D of EP1632232 B1 is a hydrate and thus not preferred for the preparation of an orally administered medicament, whereas form E of the present invention is an anhydrate, in particular a non-hydrated form, and hence especially suitable for the preparation of an orally administered medicament.

The novel crystalline form E of Rilpivirine hydrochloride of the present invention shows high solubility in aqueous systems e.g. a higher solubility than forms A and C of EP1632232 B1 and is thus especially suitable for the preparation of an orally administered medicament.

In addition the crystalline forms A and C of EP1632232 B1 are difficult to make in a reliable manner as these forms are obtained from the same solvent system. As the polymorphs A and C of Rilpivirine hydrochloride are obtainable from the same solvent system acetic acid/water, the production processes are especially critical and sensitive because the single crystalline forms are only obtainable in pure form in a quite narrow range of temperature as described in the concrete examples A.a) and A.c) of EP1632232 B1. In contrast form E of the present invention is reliably obtained by crystallization from ethanol as form E is the only crystalline form of Rilpivirine hydrochloride obtained from this solvent system.

According to example A.b) of EP1632232 B1 form B is obtained by recrystallizing Rilpivirine hydrochloride from propanone using an initial Rilpivirine hydrochloride concentration of 0.3 g/L. However, this concentration is not suitable for up-scaling as larger amounts of Rilpivirine hydrochloride would ask for tremendous solvent volumina and hence the usage of tremendously large reaction vessels. In contrast form E of the present invention is also obtained by applying higher initial Rilpivirine hydrochloride concentrations such as e.g. ≥10 g/L and is thus suitable for large scale production.

Hence, aim of the present invention is to circumvent the drawbacks of the known forms A, B, C and D of EP1632232 B1 by providing an anhydrous, in particular a non-hydrated crystalline form of Rilpivirine hydrochloride, which is obtainable in an easy and reliable manner also in large scale. In addition the novel crystalline form shows high solubility in aqueous systems making it especially suitable for the preparation of an orally administered medicament.

### SUMMARY OF THE INVENTION

The inventors of the present invention have found a novel crystalline form of Rilpivirine hydrochloride, in the following named Rilpivirine hydrochloride form E. Form E is an anhydrous, in particular a non-hydrated crystalline form and shows advantageous physical properties compared to the known forms A, B, C and D of Rilpivirine hydrochloride of EP1632232 B1 making it especially suitable for the preparation of an orally administered medicament.

Hence, in a preferred embodiment the present invention relates to a novel crystalline form of Rilpivirine hydrochloride, in the following named Rilpivirine hydrochloride form E. Form E of Rilpivirine hydrochloride can be characterized by showing an X-ray powder diffractogram comprising characteristic peaks at 2-theta angles of 8.2 ± 0.2°, 9.0 ± 0.2°, 16.7 ± 0.2°, 18.8 ± 0.2° and 24.4 ± 0.2°.

In a further preferred embodiment, the present invention relates to a process of preparing form E of Rilpivirine hydrochloride comprising recrystallizing Rilpivirine hydrochloride from ethanol.

Furthermore the present invention relates to the use of the novel crystalline form E of Rilpivirine hydrochloride for the preparation of a medicament.

In addition the present invention relates to pharmaceutical compositions comprising an effective amount of the novel crystalline form E of Rilpivirine hydrochloride and a pharmaceutically acceptable carrier and to processes of preparing the same.

Finally the present invention relates to pharmaceutical combinations comprising the novel crystalline form E of Rilpivirine hydrochloride and additional therapeutic agents.

### BRIEF DESCRIPTION OF THE DRAWINGS

Figure 1: X-ray powder diffractogram (XRPD) of Rilpivirine hydrochloride form E
Figure 2: Fourier transform infrared (FTIR) spectrum of Rilpivirine hydrochloride form E

### DETAILED DESCRIPTION OF THE INVENTION

As used herein the term "room temperature" indicates that the applied temperature is not critical and that no exact temperature value has to be kept. Usually, "room temperature" is understood to mean temperatures of about 15°C to about 25°C [see e.g. EU Pharmacopoeia 7.2, 1.2 (2011)].

In a first aspect, the present invention relates to a novel crystalline form of Rilpivirine hydrochloride (hereinafter also referred to as Rilpivirine hydrochloride form E).

Rilpivirine hydrochloride exists in two stereoisomeric forms, namely an E-isomeric form and a Z-isomeric form. Crystalline form E of Rilpivirine hydrochloride of the present invention is preferably present as the pure E-isomer, whereat pure in this context refers to an E-isomer content of at least about 95 %, more preferably of at least about 98 % and most preferably of at least about 99 %.

The chemical structure of the E-isomeric form of Rilpivirine hydrochloride is represented by the following general formula (II):

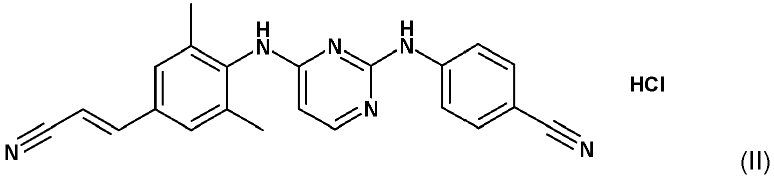

The chemical structure of the Z-isomeric form of Rilpivirine hydrochloride is represented by the following general formula (III):

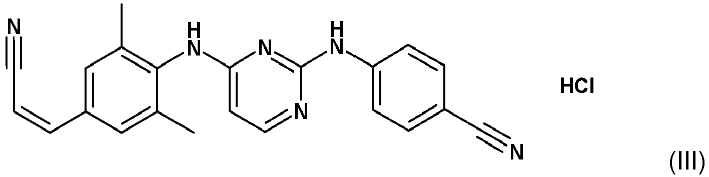

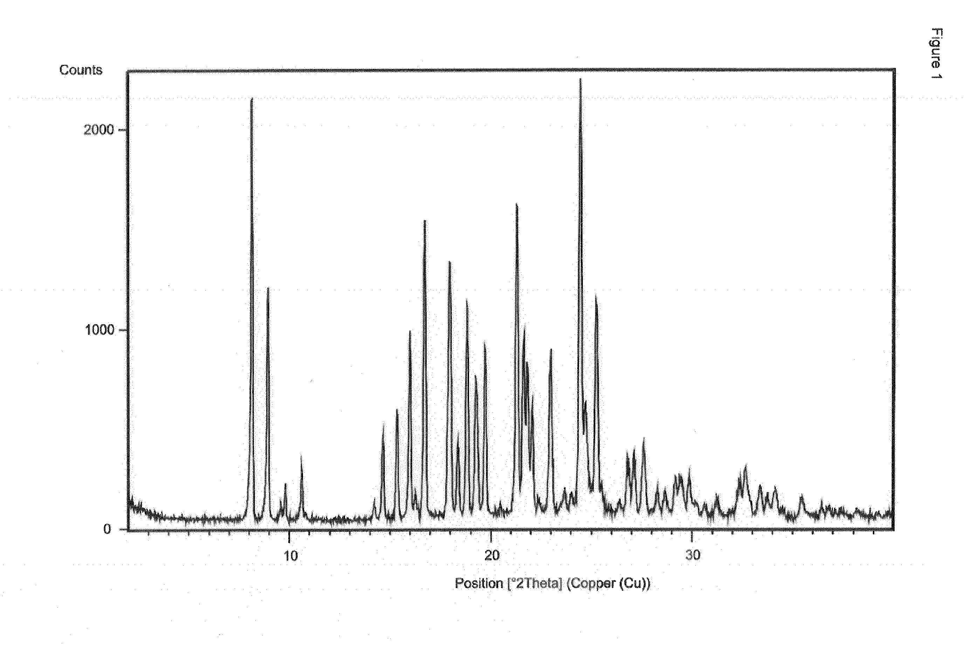

Form E of Rilpivirine hydrochloride can be characterized by showing an X-ray powder diffractogram comprising characteristic peaks at 2-theta angles of 8.2 ± 0.2°, 9.0 ± 0.2°, 10.6 ± 0.2°, 14.7 ± 0.2°, 15.4 ± 0.2°, 16.0 ± 0.2°, 16.7 ± 0.2°, 18.0 ± 0.2°, 18.4 ± 0.2°, 18.8 ± 0.2°, 19.3 ± 0.2°, 19.7 ± 0.2°, 21.3 ± 0.2°, 21.6 ± 0.2°, 21.8 ± 0.2°, 22.1 ± 0.2°, 23.0 ± 0.2°, 24.4 ± 0.2°, 24.7 ± 0.2°, 25.2 ± 0.2°, 27.1 ± 0.2°, 27.6 ± 0.2°, 29.2 ± 0.2° and 32.7 ± 0.2°. An illustrative diffractogram is displayed in figure 1.

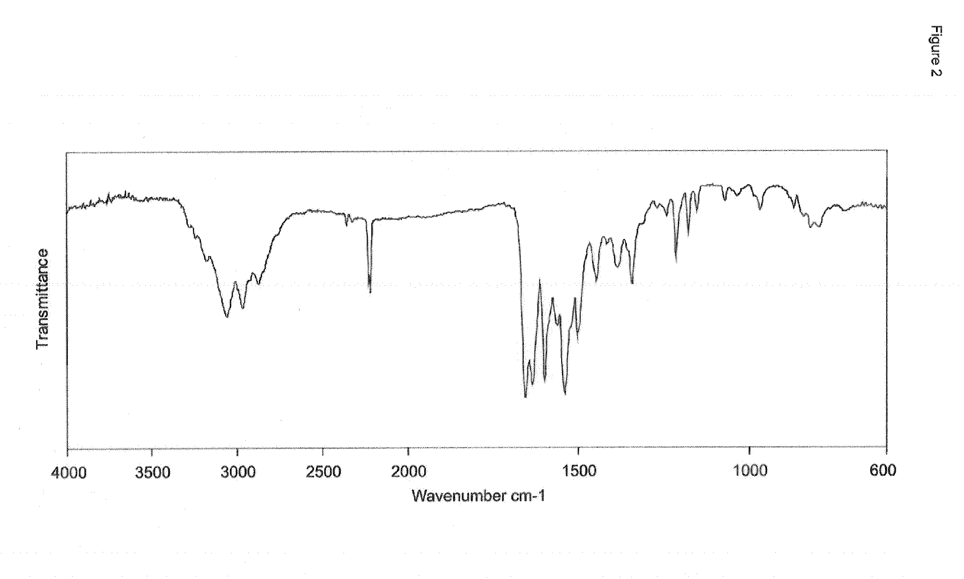

In addition form E of Rilpivirine hydrochloride can be characterized by showing an FTIR-spectrum comprising peaks at wavelengths of 3280 ± 2 cm⁻¹, 3243 ± 2 cm⁻¹, 3175 ± 2 cm⁻¹, 3057 ± 2 cm⁻¹, 2968 ± 2 cm⁻¹, 2872 ± 2 cm⁻¹, 2228 ± 2 cm⁻¹, 2218 ± 2 cm⁻¹, 1656 ± 2 cm⁻¹, 1635 ± 2 cm⁻¹, 1601 ± 2 cm⁻¹, 1562 ± 2 cm⁻¹, 1541 ± 2 cm⁻¹, 1504 ± 2 cm⁻¹, 1448 ± 2 cm⁻¹, 1417 ± 2 cm⁻¹, 1386 ± 2 cm⁻¹, 1343 ± 2 cm⁻¹, 1271 ± 2 cm⁻¹, 1243 ± 2 cm⁻¹, 1215 ± 2 cm⁻¹, 1179 ± 2 cm⁻¹, 1154 ± 2 cm⁻¹, 1072 ± 2 cm⁻¹, 1036 ± 2 cm⁻¹, 969 ± 2 cm⁻¹, 870 ± 2 cm⁻¹, 841 ± 2 cm⁻¹, 822 ± 2 cm⁻¹, 799 ± 2 cm⁻¹ and 725 ± 2 cm⁻¹. An illustrative FTIR spectrum is displayed in figure 2.

Furthermore form E can be characterized as being an anhydrate, in particular a non-hydrated form, containing about 0 % water at a relative humidity of about 0 % and about 0.8 % water at a relative humidity of about 90 %.

The present invention also relates to a process for the preparation of form E of Rilpivirine hydrochloride comprising recrystallizing Rilpivirine hydrochloride from ethanol.

In a first step a mixture of Rilpivirine hydrochloride in ethanol is provided. Any form of Rilpivirine hydrochloride may be applied e.g. amorphous Rilpivirine hydrochloride, crystalline Rilpivirine hydrochloride or a mixture of amorphous and crystalline Rilpivirine hydrochloride. Suitable crystalline forms of Rilpivirine hydrochloride are e.g. forms A, B, C and D of EP1632232 B1 or mixtures thereof.

The initial Rilpivirine hydrochloride concentration applied in the process may range from about 0.1 to 50.0 g/L, preferably from about 0.5 to 30.0 g/L, more preferably from about 1.0 to 20.0 g/L and most preferably from about 5.0 to 10.0 g/L.

The starting material is preferably dissolved upon heating the mixture to about 30 °C to reflux temperature, preferably to about 45 °C to reflux temperature, more preferably to about 60 °C to reflux temperature and most preferably to about 75 °C to reflux temperature, whereat the reflux temperature is about 78 °C. The obtained solution may optionally be filtered in order to remove any undissolved particles.

The crystallization of form E is initiated by cooling the solution to a temperature ranging from about -25 to 60 °C, preferably from about -20 to 50 °C, more preferably from about -15 to 20 °C and most preferably from about -10 to about 10 °C. A cooling rate preferably ranging from about -0.1 to -20 °C/min, more preferably from about -0.5 to -10 °C/min and most preferably from about -1 to -5 °C/min is applied.

In order to improve the yield an antisolvent may be added to the obtained suspension. Suitable antisolvents in the process of the present invention are e.g. but not limited to aliphatic C₅-C₈ hydrocarbons such as e.g. n-pentane, n-hexane, n-heptane and isooctane, mixtures of aliphatic hydrocarbons such as e.g. petroleum ether, cyclic hydrocarbons such as e.g. cyclohexane and ethers such as e.g. diethyl ether, tert-butylmethyl ether and isopropyl ether.

A preferred ethanol/antisolvent ratio (volume : volume) for the process of the present invention ranges from about 1 : 0.25 to 5, more preferably from about 1 : 0.5 to 3 and most preferably from about 1 : 1 to 2.

The antisolvent is preferably added at a temperature ranging from about -25 to 78 °C, more preferably from about -10 to 50 °C and most preferably from about 0 to 25 °C at an addition rate preferably ranging from about 1 to 500 mL/min, more preferably from about 10 to 250 mL/min and most preferably from about 20 to 100 mL/min.

The obtained crystals are then collected by any conventional methods such as filtration or centrifugation, preferably by filtration. A washing step may also be applied by rinsing the crystals with ethanol, antisolvent or a mixture of ethanol and antisolvent and/or slurrying the crystals in ethanol, antisolvent or a mixture of ethanol and antisolvent, whereat the ratio of ethanol and antisolvent is preferably the same as the ethanol/antisolvent ratio of the mother liquor.

Thereafter the crystals are dried preferably under vacuum at a temperature preferably ranging from about 25 °C to 100 °C, more preferably from about 30 °C to 80 °C and most preferably from about 40 °C to 60 °C for a time preferably ranging from about 1 to 72 hours, more preferably from about 6 to 48 hours and most preferably from about 12 to 24 hours.

The particle size of Rilpivirine hydrochloride form E obtained according to the process of the present invention typically ranges from about 10 to 100 µm determined by optical light microscopy. However, the particle size can be decreased by any conventional method such as e.g. milling or grinding. In addition the particle size can be homogenized by applying an additional sieving step. Hence, a preferred particle size of the crystalline form E of the present invention ranges from about 0.1 to 50 µm, more preferably from about 0.1 to 20 µm and most preferably from about 0.1 to 10 µm.

The bioavailability of a compound intended to be administered orally, is dependent on the solubility of the compound in aqueous systems, such as water, as well as the permeability of the compound as mentioned in EP1632232 B1. It is known to the person skilled in the art that the solubility of a crystalline solvate form in the solvent, which is incorporated in this form, is smaller than the solubility of a non-solvated form of the same compound. This means that the solubility of a hydrate - wherein water is the incorporated solvent - in water is thus smaller than that of a corresponding non-hydrated form. For example hydrates are known to be less soluble in aqueous systems than anhydrous forms of the same compound. Hence anhydrous forms of Rilpivirine hydrochloride are preferred over hydrated forms. Rilpivirine hydrochloride form D of EP1632232 B1 is a hydrate and thus not preferred for the preparation of an orally administered medicament, whereas form E of the present invention is an anhydrate, in particular a non-hydrated form, and hence especially suitable for the preparation of an orally administered medicament.

The novel crystalline form E of the present invention shows high solubility in aqueous systems e.g. a higher solubility than forms A and C of EP1632232 B1 in 0.01 N aqueous HCl and is thus especially suitable for the preparation of an orally administered medicament. The solubility data of form E of the present invention and forms A and C of EP1632232 B1 in 0.01 N aqueous HCl at 25 ± 0.1 °C are presented in table 1.

**Table 1: Solubility data in 0.01 N aqueous HCl at 25 ± 0.1 °C**

| **Form** | **Solubility [mg/mL]** |
|---|---|
| A | 0.08 |
| C | 0.10 |
| E | 0.27 |

In addition the crystalline forms A and C of EP1632232 B1 are difficult to make in a reliable manner as these forms are obtained from the same solvent system. As the polymorphs A and C of Rilpivirine hydrochloride are obtainable from the same solvent system acetic acid/water, the production processes are especially critical and sensitive because the single crystalline forms are only obtainable in pure form in a quite narrow range of temperature as described in the concrete examples A.a) and A.c) of EP1632232 B1. In contrast form E of the present invention is reliably obtained by crystallization from ethanol as form E is the only crystalline form of Rilpivirine hydrochloride obtained from this solvent system.

According to example A.b) of EP1632232 B1 form B is obtained by recrystallizing Rilpivirine hydrochloride from propanone using an initial Rilpivirine hydrochloride concentration of 0.3 g/L. However, this concentration is not suitable for up-scaling as larger amounts of Rilpivirine hydrochloride would ask for tremendous solvent volumina and hence the usage of tremendously large reaction vessels. In contrast form E of the present invention is obtained by applying higher initial Rilpivirine hydrochloride concentrations such as e.g. ≥10 g/L and is thus suitable for large scale production.

Hence, the novel crystalline form E of Rilpivirine hydrochloride of the present invention circumvents the drawbacks of the known forms A, B, C and D of EP1632232 B1 as it is obtainable in an easy and reliable manner also in large scale. In addition the novel crystalline form shows high solubility in aqueous systems e.g. higher solubility in 0.01 N aqueous HCl compared to forms A and C of EP1632232 B1 making it especially suitable for the preparation of an orally administered medicament.

Hence, form E of Rilpivirine hydrochloride is the most favored form to be used in an antiretroviral pharmaceutical composition and may advantageously be employed in various pharmaceutical formulations for use in the treatment of HIV-1 infection. The present invention therefore also relates to a pharmaceutical composition which comprises Rilpivirine hydrochloride form E as described above and a pharmaceutically acceptable carrier.

Preferably, the present invention relates to pharmaceutical compositions, wherein more than 95 % of Rilpivirine hydrochloride is stably present as Rilpivirine hydrochloride form E, more preferably wherein Rilpivirine hydrochloride form E is the only detectable crystalline form of Rilpivirine hydrochloride. The absence of other crystalline forms of Rilpivirine hydrochloride, such as forms A, B, C and D of EP1632232 B1 can be tested by comparing an XRPD taken of any crystalline Rilpivirine hydrochloride with the XRPD of form E as obtained from example 1 and shown in figure 1, which for this comparison is to be taken as an XRPD of 100 % form E.

"Stably present" as defined herein means that even after storage of the pharmaceutical composition for 180 days, and preferably even after storage for 3 years, the crystalline form of Rilpivirine hydrochloride designated as Rilpivirine hydrochloride form E initially comprised in the pharmaceutical composition is still present as Rilpivirine hydrochloride form E after storage for the indicated period.

The pharmaceutical compositions of the invention comprising Rilpivirine hydrochloride form E may further comprise one or more pharmaceutically acceptable excipients. Such excipients are preferably selected from the group consisting of fillers, sweeteners, buffering agents, glidants, flowing agents, flavouring agents, lubricants, preservatives, surfactants, wetting agents, binders, disintegrants and thickeners. Other excipients known in the field of pharmaceutical compositions may also be used. Furthermore, the pharmaceutical composition may comprise a combination of two or more excipients also within one of the members of the above mentioned group.

Examples of suitable excipients for pharmaceutical compositions of the invention comprising Rilpivirine hydrochloride form E are given e.g. in EP1632232 B1, which is herein incorporated by reference, in paragraphs [0065] to [0080].

Paragraphs [0065] to [0072] of EP1632232 B1 disclose examples of binders for the pharmaceutical compositions of the present invention comprising Rilpivirine hydrochloride form E. The preferred binders, which can also be used for the pharmaceutical compositions of the present invention, comprise e.g. alkylcelluloses such as methylcellulose, hydroxyalkylcelluloses such as hydroxymethylcellulose, hydroxyethylcellulose, hydroxypropylcellulose and hydroxybutylcellulose, hydroxyalkylalkylcelluloses such as hydroxyethylmethylcellulose and hydroxypropylmethylcellulose, carboxyalkylcelluoses such as carboxymethylcellulose, alkali metal salts of carboxyalkylcelluloses such as sodium carboxymethylcellulose, carboxyalkylalkylcelluloses such as carboxymethylethylcellulose, carboxyalkylcellulose esters, starches such as starch 1551, pectins such as sodium carboxymethylamylopectin, chitin derivatives such as chitosan, heparin and heparinoids, polysaccharides such as alginic acid, alkali metal and ammonium salts thereof, carrageenans, galactomannans, tragacanth, agar-agar, gum arabic, guar gum and xanthan gum, polyacrylic acids and the salts thereof, polymethacrylic acids and the salts thereof, methacrylate copolymers, polyvinylalcohol, polyvinylpyrrolidone, copolymers of polyvinylpyrrolidone with vinyl acetate, polyalkylene oxides such as polyethylene oxide and polypropylene oxide and copolymers of ethylene oxide and propylene oxide, e.g. poloxamers and poloxamines, copovidone, whereat starch, polyvinylpyrrolidone, a cellulose ether such as PVP K29-32, PVP K90, methyl cellulose, hydroxypropylcellulose, hydroxyethyl methylcellulose or hydroxypropyl methylcellulose (HPMC) are preferred.

Paragraph [0075] of EP1632232 B1 discloses examples of suitable diluents for the pharmaceutical compositions of the present invention comprising Rilpivirine hydrochloride form E. The preferred diluents, which can also be used for the pharmaceutical compositions of the present invention, comprise e.g. calcium carbonate, dibasic calcium phosphate, dibasic calcium phosphate dihydrate, tribasic calcium phosphate, calcium sulphate, microcrystalline cellulose including silicified microcrystalline cellulose, powdered cellulose, dextrates, dextrin, dextrose excipient, fructose, kaolin, lactitol, lactose anhydrous, lactose monohydrate, mannitol, sorbitol, starch, pregelatinized starch, sodium chloride, sucrose, compressible sugar, confectioner's sugar, a spray-dried mixture of lactose monohydrate and microcrystalline cellulose (75:25), commercially available as Microcelac®, a co-processed spray-dried mixture of microcrystalline cellulose and colloidal silicon dioxide (98:2), commercially available as Prosolv®, whereat lactose monohydrate, microcrystalline cellulose or silicified microcrystalline cellulose are preferred.

Paragraph [0076] of EP1632232 B1 discloses examples of glidants for the pharmaceutical compositions of the present invention comprising Rilpivirine hydrochloride form E. The preferred glidants, which can also be used for the pharmaceutical compositions of the present invention comprise talc, colloidal silicon dioxide, starch and magnesium stearate, whereat magnesium stearate is preferred.

Paragraph [0078] of EP1632232 B1 discloses examples of disintegrants for the pharmaceutical compositions of the present invention comprising Rilpivirine hydrochloride form E. The preferred disintegrants, which can also be used for the pharmaceutical compositions of the present invention, comprise starch, ion exchange resins, e.g. Amberlite, cross-linked polyvinylpyrrolidone, modified cellulose gum, e.g croscarmellose sodium, sodium starch glycolate, sodium carboxymethylcellulose, sodium dodecyl sulphate, modified corn starch, microcrystalline cellulose, magnesium aluminium silicate, alginic acid, alginate and powdered cellulose, whereat croscarmellose sodium is preferred.

Paragraph [0079] of EP1632232 B1 discloses examples of lubricants for the pharmaceutical compositions of the present invention comprising Rilpivirine hydrochloride form E. The preferred lubricants, which can also be used for the pharmaceutical compositions of the present invention, are e.g. magnesium stearate, calcium stearate, stearic acid, talc, polyethylene glycol, sodium lauryl sulphate and magnesium lauryl sulphate, whereat magnesium stearate is preferred.

In addition the pharmaceutical compositions of the present invention comprising Rilpivirine hydrochloride form E may comprise other optional excipients such as, for example, flavors, sweeteners and colors.

Paragraph [0083] of EP1632232 B1 discloses examples of film coatings for the pharmaceutical compositions of the present invention comprising Rilpivirine hydrochloride form E. The film coatings, which can also be used for the pharmaceutical compositions of the present invention, are preferably immediate release film coatings comprising a film-forming polymer and optionally a plasticizer or a pigment. An example for a suitable film-forming polymer is hydroxypropyl methylcellulose, an example for a suitable plasticizer is polyethyleneglycol, e.g. macrogol 3000 or 6000, or triacetin and an example for a suitable pigment is titanium dioxide.

A preferred pharmaceutical composition of the present invention comprises a tablet core comprising Rilpivirine hydrochloride form E of the present invention, croscarmellose sodium, magnesium stearate, lactose monohydrate, povidone K30, polysorbate 20, and silicified microcrystalline cellulose and a tablet coating comprising hypromellose 2910 6 mPa.s, lactose monohydrate, PEG 3000, titanium dioxide and triacetin.

Examples of suitable processes for the preparation of the pharmaceutical compositions of the present invention are given e.g. in EP1632232 B1, which is herein incorporated by reference, in paragraphs [0084] to [0086], wherein it is to be understood that whenever the term compound of formula (I), (Ia) or (I-b) or active ingredient is used in EP1632232 B1 an equivalent amount of Rilpivirine hydrochloride form E of the present invention is to be used.

Concrete examples for the production of formulations of the present invention are given e.g. in EP1632232 B1, paragraphs [0120] to [0137]. These examples can be repeated using Rilpivirine hydrochloride form E of the present invention.

Formulations of the present invention typically comprise 1 to 250 mg of Rilpivirine hydrochloride form E, whereat the preferred dosage is 25 mg (calculated as Rilpivirine base).

In a further embodiment the present invention relates to a pharmaceutical combination comprising an effective amount of Rilpivirine hydrochloride form E of the present invention and additional therapeutic agents such as e.g. anti-virals, antibiotics, immunomodulators or vaccines for the treatment of viral infections. Preferably the additional therapeutic agents are chosen from e.g. emtricitabine, tenofovir, abacavir, lamivudine, efavirenz, ritonavir, atazanavir, raltegravir, darunavir, fosamprenavir, lopinavir, telaprevir, boceprevir and/or zidovudine, most preferably emtricitabine, tenofovir, abacavir and/or lamivudine are used for the pharmaceutical combinations.

Other objects, features, advantages and aspects of the present invention will become apparent to those of skill from the following description. It should be understood, however, that the description and the following specific examples, while indicating preferred embodiments of the invention, are given by way of illustration only. Various changes and modifications within the spirit and scope of the disclosed invention will become readily apparent to those skilled in the art from reading the description and the other parts of the present disclosure.

### EXAMPLES

The X-ray powder diffractogram (XRPD) was obtained with an X'Pert PRO diffractometer (PANalytical, Almelo, The Netherlands) equipped with a theta/theta coupled goniometer in transmission geometry, programmable XYZ stage with well plate holder, Cu-Kα1,2 radiation source (wavelength 0.15419 nm) with a focussing mirror, a 0.5° divergence slit, a 0.02° soller slit collimator and a 1 °anti-scattering slit on the incident beam side, a 2 mm anti-scattering slit, a 0.02° soller slit collimator and a Nickel filter on the diffracted beam side and a solid state PIX'cel detector. The pattern was recorded at a tube voltage of 40 kV, tube current of 40 mA, applying a stepsize of 0.013° 2-theta with 40 s per step in the angular range of 2° to 40° 2-theta. A typical precision of the 2-theta values is in the range of about ± 0.2° 2-theta. Thus a diffraction peak that appears at 5.0° 2-theta can appear between 4.8 and 5.2° 2-theta on most X-ray diffractometers under standard conditions.

The Fourier transform infrared (FTIR) spectrum was recorded with a Bruker IFS 25 spectrometer (Bruker GmbH, Karlsruhe, D) in the spectral range from 4000 to 600 cm⁻¹ with a resolution of 2 cm⁻¹ (64 scans). The sample was prepared on a ZnSe disk using the Bruker IR microscope I, with 15x-Cassegrain-objectives. A typical precision of the wavenumber values is in the range of about ± 2 cm ⁻¹. Thus, an infrared peak that appears at 1716 cm⁻¹ can appear between 1714 and 1718 cm⁻¹.

### Example 1: Preparation of crystalline form E of Rilpivirine hydrochloride

A suspension of 500 mg Rilpivirine hydrochloride in 50 mL ethanol was heated to reflux, whereat a clear solution was obtained. The hot solution was filtered and cooled in an ice bath, whereat crystallization was observed during cooling. The obtained suspension was further stirred for 15 hours at room temperature before the crystals were collected by filtration and dried at 80 °C under vacuum for 15 hours. 320 mg of crystalline form E of Rilpivirine hydrochloride were received.

**Table 2: XRPD angles 2-theta, relative intensities of crystalline form E of Rilpivirine hydrochloride**

| **angle** | **relative intensity** | **angle** | **relative intensity** |
|---|---|---|---|
| **[2-theta]** | **[%]** | **[2-theta]** | **[%]** |
| 8.2 | 96 | 21.3 | 71 |
| 9.0 | 53 | 21.6 | 40 |
| 10.6 | 12 | 21.8 | 34 |
| 14.7 | 20 | 22.1 | 26 |
| 15.4 | 24 | 23.0 | 39 |
| 16.0 | 43 | 24.4 | 100 |
| 16.7 | 68 | 24.7 | 26 |
| 18.0 | 58 | 25.2 | 48 |
| 18.4 | 17 | 27.1 | 14 |
| 18.8 | 49 | 27.6 | 17 |
| 19.3 | 31 | 29.2 | 10 |
| 19.7 | 39 | 32.7 | 11 |

**Table 3: FTIR peaks of crystalline form E of Rilpivirine hydrochloride**

| **wavelength [cm⁻¹]** | | |
|---|---|---|
| 3280 | 1562 | 1154 |
| 3243 | 1541 | 1072 |
| 3175 | 1504 | 1036 |
| 3057 | 1448 | 969 |
| 2968 | 1417 | 870 |
| 2872 | 1386 | 841 |
| 2228 | 1343 | 822 |
| 2218 | 1271 | 799 |
| 1656 | 1243 | 725 |
| 1635 | 1215 | |
| 1601 | 1179 | |

### Example 2: Preparation of crystalline form E of Rilpivirine hydrochloride

A suspension of 200 mg Rilpivirine hydrochloride in 24 mL ethanol was heated to reflux, whereat a clear solution was obtained. The hot solution was filtered and cooled in an ice bath, whereat crystallization was observed during cooling. To the obtained suspension 24 mL n-heptane were added and the suspension was further stirred at room temperature for 15 hours. The obtained crystals were collected by filtration and dried at 80 °C under vacuum for 15 hours. 162 mg of crystalline form E of Rilpivirine hydrochloride were received.

### Example 3: Preparation of crystalline form E of Rilpivirine hydrochloride

A suspension of 200 mg Rilpivirine hydrochloride in 24 mL ethanol was heated to reflux, whereat a clear solution was obtained. The hot solution was filtered and cooled to room temperature, whereat crystallization was observed after about 3 hours. To the obtained suspension 24 mL n-heptane were added and the suspension was further stirred at room temperature for 15 hours. The obtained crystals were collected by filtration and dried at 80 °C under vacuum for 15 hours. 150 mg of crystalline form E of Rilpivirine hydrochloride were received.

### Example 4: Solubility determination

Suspensions of forms A, C and E in 0.01 N aqueous HCl were stirred in closed vessels with a diameter of 25 mm in a thermostatic water bath at 25 ± 0.1 °C. Before the solute was added the solvent was equilibrated to the temperature. Three samples of each modification were taken with Swinnex filter holders attached to volumetric pipettes (2 mL) via a small glass tube after 30 min. They were sucked through Millipore membrane filters (0.45 µm) and 2 mL of the clear solution were poured in vessels which had been dried and tared before. The hydrochloric acid was evaporated in a drying chamber at 60 °C for approximately 15 h before the temperature was further increased to 80 °C for approximately 3 h. To remove residue water, the vessels were subsequently dried at 110 °C and 60 mbar for approximately 3 h. Afterwards the vials with the residue were cooled to room temperature in an exsiccator and weighed on an analysis balance (Mettler AT250). The difference in weight to the tare weight was then measured to determine the solubilities.

## Claims

1. Crystalline form of Rilpivirine hydrochloride having an X-ray powder diffraction pattern comprising peaks at 2- theta angles of 8.2 ± 0.2°, 9.0 ± 0.2°, 16.7 ± 0.2°, 18.8 ± 0.2° and 24.4 ± 0.2°, preferably **characterized by** an X-ray powder diffraction pattern essentially in accordance with figure 1.

2. The crystalline form of Rilpivirine hydrochloride according to claim 1 **characterized by** an infrared spectrum comprising peaks at wavenumbers of 3057 ± 2 cm⁻¹, 2968 ± 2 cm⁻¹, 2228 ± 2 cm⁻¹, 2218 ± 2 cm⁻¹ and 1656 ± 2 cm⁻¹, preferably **characterized by** an infrared spectrum essentially in accordance with figure 2.

3. The crystalline form of Rilpivirine hydrochloride according to any of claims 1 to 2 **characterized by** a water content of about 0 % at about 0 % relative humidity and about 0.8 % at about 90 % relative humidity.

4. The crystalline form of Rilpivirine hydrochloride according to any of claims 1 to 3 characterized as being non-hydrated form.

5. The crystalline form of Rilpivirine hydrochloride according to any of claims 1 to 4 having a particle size of 0.1 to 50 µm.

6. The crystalline form of Rilpivirine hydrochloride according to any of claims 1 to 4 having a particle size of 0.1 to 25 µm.

7. The crystalline form of Rilpivirine hydrochloride according to any of claims 1 to 4 having a particle size of 0.1 to 15 µm.

8. A method for the preparation of the crystalline form of Rilpivirine hydrochloride according to any one of claims 1 to 7 comprising the step of recrystallizing Rilpivirine hydrochloride from ethanol.

9. A pharmaceutical composition comprising a crystalline form of Rilpivirine hydrochloride according to any one of claims 1 to 7, further comprising at least one pharmaceutically acceptable excipient.

10. The pharmaceutical composition according to claim 9, which is an oral dosage form, in particular a capsule or tablet.

11. A tablet according to claim 10 comprising a tablet core comprising the crystalline form of Rilpivirine hydrochloride according to any of claims 1 to 7, croscarmellose sodium, magnesium stearate, lactose monohydrate, povidone K30, polysorbate 20 and silicified microcrystalline cellulose and a tablet coating comprising hypromellose 2910 6 mPa.s, lactose monohydrate, PEG 3000, titanium dioxide and triacetin.

12. Use of a crystalline form of Rilpivirine hydrochloride according to any one of claims 1 to 7 for the production of a pharmaceutical composition.

13. The pharmaceutical composition as defined in any of the claims 9 to 11 or as obtained by the use according to claim 12 for use in the treatment of HIV-1 infections.

14. A process for preparing a pharmaceutical composition comprising the crystalline form of Rilpivirine hydrochloride according to any of claims 1 to 7, comprising the steps of carrying out a process according to claim 8 and mixing the crystalline form of Rilpivirine hydrochloride according to any of claims 1 to 7 with at least one pharmaceutically acceptable excipient.

15. A pharmaceutical combination comprising an effective amount of the crystalline form of Rilpivirine hydrochloride according to any of claims 1 to 7 and additional therapeutic agents such as anti-virals, antibiotics, immunomodulators or vaccines for the treatment of viral infections.
